# EUROPEAN PATENT APPLICATION

(11) **EP 4 674 645 A1**
(43) Date of publication of application: **07.01.2026**
(21) Application number: 24186758.9
(22) Date of filing: 05.07.2024
(51) Int. Cl.: B60C 11/24, B60C 19/00, G01N 33/44, G06K 19/077, B29D 30/00

(54) **TYRE PLASTICIZER MIGRATION MONITORING**

(71) Applicant: Bridgestone Europe NV/SA, 1930 Zaventem (BE)
(72) Inventor: Lombardi, Roberto, 00128 Roma (IT)
(74) Representative: Marks & Clerk LLP

(57) **Abstract**

A tyre T has a higher plasticizer content region and a lower plasticizer content region, and the tyre T has integrated in it an RF transmitter and a plasticizer absorbing material configured to absorb migrated plasticizer. The relative dielectric constant of the plasticizer absorbing material when the tyre is in a cured state is ≥6.5. As plasticizer is absorbed, the relative dielectric constant of the plasticizer absorbing material changes which in turn changes the RF signal strength from the RF transmitter. By providing a tyre with a relative dielectric constant of the plasticizer absorbing material in this range, migration of the plasticizer can be easily monitored using an RF receiver.

## Description

### Technical Field

The present invention relates to monitoring plasticizer migration in a tyre.

### Background

The usage of plasticizers (or "oils") in rubber compounds for tyre applications is widespread, not only to improve compound processability during the manufacturing stage but also to impart special characteristics to tread compounds such as wet braking or snow braking performance (i.e. the so called winter performance) in tyre products.

On the other hand it is well known that plasticizers, especially those having low Tg (glass transition temperature) or low MW (molecular weight), are prone to migrate from high concentration area (such as in tread compounds) to lower concentration area (such as in carcass compounds, especially in the crown area) according to the diffusion phenomenon that is quantitatively described by physical models such as Fick's laws. The speed of this phenomenon is further accelerated by temperature.

The "donor" compound (e.g. the tyre tread) experiences a progressive reduction in plasticizers content during the whole tyre life (both during storage in warehouses and during market exposure) and a consequent worsening in plasticizers-controlled performance such as wet braking and snow braking with respect to initial design criteria. At the same time, the plasticizer "recipient" compounds (e.g. crown area compounds such as the base compound, the cap ply skim (CPS) and the tread ply skim (TPS)) experience a progressive increase in plasticizer content, leading to a time-dependent deterioration of some characteristics such as ultimate mechanical properties (related to tyre durability).

Considering the importance of abovementioned phenomena, there is a desire for tire and compound designers to measure the progress of plasticizers diffusion in tire components.

Currently, according to conventional testing methods, the amount of plasticizers migrated into a given tire component can be physically measured only through destructive methods.

The present invention aims to mitigate at least one of the drawbacks of the conventional testing methods.

### Summary of the Invention

A first aspect of the present invention provides a tyre having a higher plasticizer content region and a lower plasticizer content region, the tyre having integrated in it an RF transmitter and a plasticizer absorbing material configured to absorb migrated plasticizer, wherein the relative dielectric constant of the plasticizer absorbing material when the tyre is in a cured state is ≥6.5.

By providing a tyre with a relative dielectric constant of the plasticizer absorbing material in this range at operating frequency of the RF transmitter, migration of the plasticizer can be easily monitored using a method or apparatus according to another aspect of the present invention. The operating frequency may be, for example, above 10 MHz and/or below 1600 MHz, or above 400 MHz and/or below 1000 MHz.

As the plasticizer absorbing material has a lower plasticizer content than the higher plasticizer content region of the tyre, the plasticizer can migrate from the higher plasticizer content region and be absorbed into the plasticizer absorbing material which will change the relative dielectric constant of the plasticizer absorbing material.

The RF transmitter and/or the plasticizer absorbing material may be embedded within the tyre, or may be adhered to a surface of the tyre, for example, to the inner surface of the tyre.

The RF transmitter and/or the plasticizer absorbing material may be in contact with or embedded in the higher plasticizer content region and/or the lower plasticizer content region.

In one example, the relative dielectric constant is in this range when the tyre has just been cured. Here, the relative dielectric constant is ≥6.5 initially, and the figure drops as plasticizer is absorbed. In any prior art tyres in which a relative dielectric constant is lower than 6.5, the relative dielectric constant will drop too as plasticizer is absorbed, and therefore the relative dielectric constant will never rise into the range of ≥6.5. Typically, when the tyre has just been cured, the plasticizer absorbing material (or "coating gum") will not have had time to absorb much plasticizer.

Optionally, the RF transmitter is an RFID tag.

Optionally, the relative dielectric constant of the plasticizer absorbing material when the tyre is in a cured state ≥7, ≥ 8 or ≥9.

Optionally, for each unit% of adsorbed plasticizer amount, the plasticizer absorbing material relative dielectric constant changes in an absolute value range of from 0.1 to 2.

A second aspect of the present invention provides a tyre having a higher plasticizer content region and a lower plasticizer content region, the tyre having integrated in it an RF transmitter and a plasticizer absorbing material configured to absorb migrated plasticizer, wherein, for each unit% of adsorbed plasticizer amount, the relative dielectric constant of the plasticizer absorbing material changes in an absolute value range of from 0.1 to 2.

By providing a tyre with a relative dielectric constant of the plasticizer absorbing material that changes in this range at operating frequency of the RF transmitter, migration of the plasticizer can be easily monitored using a method or apparatus according to another aspect of the present invention. The operating frequency may be, for example, above 10 MHz and/or below 1600 MHz, or above 400 MHz and/or below 1000 MHz.

As the plasticizer absorbing material has a lower plasticizer content than the higher plasticizer content region of the tyre, the plasticizer can migrate from the higher plasticizer content region and be absorbed into the plasticizer absorbing material which will change the relative dielectric constant of the plasticizer absorbing material.

Optionally, the plasticizer absorbing material coats the RF transmitter.

Optionally, the RF transmitter is an RFID tag.

In one example, the relative dielectric constant changes in this range when the tyre has just been cured.

Optionally, for each unit% of adsorbed plasticizer amount, the plasticizer absorbing material relative dielectric constant changes in an absolute value range of from 0.15 to 2; 0.2 to 2; 0.5 to 2; 1 to 2; or 1.5 to 2.

Optionally, the relative dielectric constant of the plasticizer absorbing material when the tyre is in a cured state is ≥6.5.

Optionally, the plasticizer absorbing material coats at least part of the RF transmitter.

It is not necessary for the plasticizer absorbing material (PAM) to be in contact with the RF transmitter because the RF signal can still travel through the PAM even when the PAM is spaced apart from the RF transmitter. However, it may be advantageous for the PAM to be located close to the RF transmitter (for example to at least partly coat the RF transmitter) as that may increase the likelihood of the signal passing through the PAM.

Optionally, the relative dielectric constant is in this range or changes in this range at a frequency from 10 MHz to 1600 MHz, preferably at a frequency from 400 MHz to 1000 MHz.

Optionally, the RF transmitter and/or plasticizer absorbing material are located either a) between a radially outermost tread ply of the tyre and the tread outer surface while being located axially within the width of a tread ply, or b) axially outside the width of a tread ply and within a buttress area of the tyre and between a radially outermost body ply of the tyre and the tyre outer surface.

Optionally, the plasticizer absorbing material comprises:
an elastomer present in a total amount of from 40 to 80 wt% of the plasticizer absorbing material, wherein the elastomer comprises natural rubber and one or more of butadiene rubber and styrene-butadiene rubber; and,
a reinforcing filler component present in a total amount of from 20 to 40 wt% of the plasticizer absorbing material, wherein the reinforcing filler component comprises a carbon black, preferably one or more carbon blacks having a surface area of the carbon black may be from 30 to 170 g/kg as measured by the Iodine Adsorption Number determined according to ASTM D 1510-16 and/or an Oil Absorption Number (OAN) of from 60 to 180 cm³/100 g (10⁻⁵ m³/kg) as determined according to ASTM D2414.

A third aspect of the present invention provides an RF device for embedding within a tyre, the RF device comprising an RF transmitter and a plasticizer absorbing material coating at least part of the RF transmitter for absorbing migrated plasticizer from a tyre, wherein the relative dielectric constant of the plasticizer absorbing material is ≥6.5, and/or wherein, for each unit% of adsorbed plasticizer amount, the relative dielectric constant of the plasticizer absorbing material changes in an absolute value range of from 0.1 to 2.

A fourth aspect of the present invention provides a method of determining plasticizer migration in a tyre, the tyre having a higher plasticizer content region and a lower plasticizer content region and an RF transmitter integrated in the tyre, the method comprising measuring, using an RF receiver, an RF signal strength from the RF transmitter, or measuring, using an RF receiver, an RF signal strength from the RF transmitter and acquiring information on the position at which the RF signal strength was measured; and based on the measured signal strength or the position at which the RF signal strength was measured, determining a corresponding value for plasticizer migration using information on how the signal strength or measured position varies with plasticizer migration for that tyre type.

A fifth aspect of the present invention provides a method of determining plasticizer migration in a tyre, the tyre having a higher plasticizer content region and a lower plasticizer content region and an RF transmitter integrated in the tyre, the method comprising acquiring information on an RF signal strength measured at a first time, or information on an RF signal strength measured at a first time and the position at which the RF signal strength was measured; measuring using an RF receiver an RF signal strength from the RF transmitter at a second time, or measuring using an RF receiver an RF signal strength from the RF transmitter at a second time and acquiring information on the position at which the RF signal strength was measured, and comparing the signal strengths or the positions where the measurements are taken at the first time and the second time, to give a value indicative of plasticizer migration in the tyre at the second time.

The comparing may be done by calculating the change in signal strengths or the change in positions where the measurements are taken. The comparing may be done by calculating the ratio of the signal strengths or the ratio of the positions where the measurements are taken.

Optionally, the method further comprises comparing the value indicative of plasticizer migration with a threshold value and, if the value is greater than or less than the threshold value, determining that the tyre has deteriorated beyond an acceptable level.

The positions at which the RF signal strength was measured at the first and second times may be maximum distances at which an RF signal can be detected.

The RF transmitter may be active or passive.

The RF transmitter may be configured to transmit in a frequency range of 400MHz to 1400MHz, 400MHz to 1000MHz, 400MHz to 800MHz or 400MHz to 600MHz. In one embodiment, the RF transmitter may be configured to transmit in a frequency range of about 475-550MHz.

A sixth aspect of the present invention provides a tyre condition monitoring apparatus, comprising an RF receiver; and a computer system containing a processor and a memory storing program instructions operative to cause the computer to execute the steps of the method of the fourth or fifth aspect.

The tyre condition monitoring apparatus may comprise an audio or visual output device for indicating to a user an output of the method or information based on the output.

The tyre condition monitoring apparatus may comprise a display unit for displaying an output of the method or information based on the output.

The tyre condition monitoring apparatus may comprise a memory for storing an output of the method or information based on the output.

Optionally, the computer system is provided by the ECU of a vehicle.

The computer system may be provided by a handheld device, such as a smartphone. The smartphone may include a display unit for displaying the output information. The smartphone may include the RF transmitter.

The tyre may be mounted on the vehicle. The RF transmitter may be mounted on the vehicle.

The tyre condition monitoring apparatus may comprise a server configured to communicate wirelessly with the handheld device and/or ECU of a vehicle, and configured to compare the signal strengths or the positions where the measurements are taken.

A seventh aspect of the present invention provides a tyre condition monitoring system comprising a tyre condition monitoring apparatus according to the sixth aspect and a tyre, wherein the tyre has a higher plasticizer content region and a lower plasticizer content region, and an RF transmitter integrated in the tyre.

Optionally, the tyre is according to the first or second aspect.

An eighth aspect of the present invention provides a computer program product comprising instructions, which, when the program is executed by a computer, cause the computer to implement a method according to the fourth or fifth aspect.

The optional features of each aspect of the invention are equally applicable to each other aspect of the invention.

### Brief Description of the Drawings

Preferred embodiments of the invention will now be described, purely by way of example, with reference to the drawings in which:
Fig. 1 provides an explanation of the principle behind the invention;
Fig. 2 shows two graphs illustrating the way different plasticizer absorbing materials are altered by the same plasticizer;
Fig. 3 is a graph illustrating how the relative dielectric constant of a particular plasticizer absorbing material varies according to frequency of the RF signal for different absorbed plasticizer contents;
Fig. 4 is a graph illustrating how, for a particular plasticizer absorbing material, the RF signal reading distance varies according to the amount of absorbed plasticizer;
Fig. 5 is a cross-section of a tyre along an axial-radial plane indicating within the dashed line the preferred positions for locating the RFID tag and plasticizer absorbing material;
Fig. 6 is a flowchart illustrating a first example method of using the RFID tag and plasticizer absorbing material to determine plasticizer migration in the tyre;
Fig. 7 is a flowchart illustrating a second example method of using the RFID tag and plasticizer absorbing material to determine plasticizer migration in the tyre;
Fig. 8 is a flowchart showing how the maximum distance at which an RF signal can be detected may be measured.
Fig. 9 is a block diagram showing a technical architecture of a server X; and
Fig. 10 is a block diagram showing a technical architecture of a communication device 1.

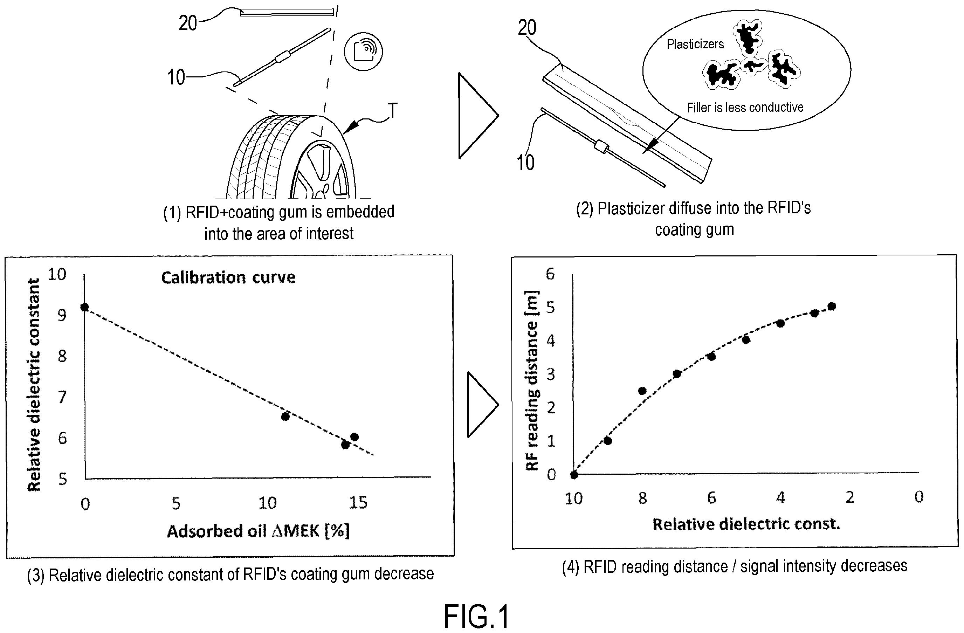

The present invention exploits the fact that in rubber compounds the dielectric properties are sensitive to changes in carbon black filler network. Since the migration of plasticizers increases the filler-filler distance, also the relative dielectric constant (a dimensionless quantity which is also known as the relative permittivity) of a given rubber compound decreases accordingly. At same time, since the reading distance of tyre-embedded sensors such as an RFID tag increases as long as the relative dielectric constant of their coating gum (also referred to herein as a plasticizer absorbing material) decreases, it is intuitive that an RFID tag can be used as a sensing device for oil migration given that the influence of plasticizer on its plasticizer absorbing material dielectric response is known (see Fig. 1 for an explanation of the invention sensing principle).

Referring to Fig. 1:
In (1), an RFID tag 10 with a plasticizer absorbing material 20 (whose dielectric response to plasticizer absorption is known) is embedded into a tyre T in the area of interest, that is to say, in the area in which it is desired to monitor the plasticizer migration. In another embodiment, the RFID tag 10 and with the plasticizer absorbing material 20 are adhered to the inner surface of the tyre T.
In (2), the plasticizer diffuses into the RFID tag 10's plasticizer absorbing material 20 over time, and this diffusion reflects the plasticizer's diffusion from a higher plasticizer content region of the tyre (for example, the tread) to a lower plasticizer content region (for example, the carcass).
In (3), the graph shows that as the amount of plasticizer (oil) absorbed by the plasticizer absorbing material increases, the relative dielectric constant of the RFID tag 10's plasticizer absorbing material 20 decreases.
In (4), the graph shows that as the relative dielectric constant of the RFID tag 10's plasticizer absorbing material 20 decreases, the maximum reading distance of the RFID tag 10 increases. This is because the plasticizer increases the filler-filler distance in the plasticizer absorbing material 20, which decreases the relative dielectric constant (the ability of the plasticizer absorbing material 20 to absorb RF signals) and thus the reading distance increases because there is less blocking effect on the signal.

As noted above, before embedding the RFID tag and plasticizer absorbing material into the tyre it is preferred to determine the dielectric response of the plasticizer absorbing material to the particular plasticizer used in the tyre. Different plasticizers may behave differently in altering the relative dielectric constant of a given plasticizer absorbing material. Similarly, different plasticizer absorbing materials may be altered differently by the same plasticizer (because of the different carbon black contents of the plasticizer absorbing materials).

On the other hand, the amount of carbon black in the components of the tyre other than the plasticizer absorbing material is believed not to have a significant effect on the reading distance or signal strength of the RFID tag.

The plasticizer absorbing material of the present invention may comprise (or be prepared from or using):
an elastomer; and,
a reinforcing filler component.

The elastomer is not particularly limited and may be any of the elastomers known to the skilled person. The elastomer may be present in a total amount of at least 40 wt% of the plasticizer absorbing material, preferably from 40 to 80 wt%, more preferably from 55 to 65 wt% of the plasticizer absorbing material, for example 55, 60, 65, 70, 75, or 80 wt% of the plasticizer absorbing material. The elastomer may be a diene rubber, such as one or more selected from the group consisting of natural rubber, synthetic polyisoprene, and diene rubber co-polymers. Preferably, the elastomer is one or more selected from the group consisting of natural rubber, butadiene rubber and styrene-butadiene rubber. In particularly preferred embodiments, the elastomer comprises natural rubber and one or more of butadiene rubber and styrene-butadiene rubber. In these particularly preferred embodiments, the amount of natural rubber may be less than 60 wt% of the plasticizer absorbing material, preferably from 35 to 60 wt%, more preferably from 40 to 55 wt% of the plasticizer absorbing material, for example 40, 45, 50 or 55 wt% of the plasticizer absorbing material, and the amount of butadiene rubber or styrene-butadiene rubber, if present, may be from 10 to 30 wt% of the plasticizer absorbing material, preferably from 15 to 25 wt%, for example 15, 20, or 25 wt% of the plasticizer absorbing material.

The reinforcing filler component may comprise a carbon black. The total amount of carbon black may be from 5 to 40 wt% of the plasticizer absorbing material, preferably from 20 to 40 wt%, more preferably from 25 to 35 wt%, for example 25, 26, 27, 28, 29, 30, 31, 32, 33, 34, or 35 wt% of the plasticizer absorbing material. The carbon black may be a carcass grade or tread grade carbon black. The surface area of the carbon black may be from 30 to 170 g/kg as measured by the Iodine Adsorption Number determined according to ASTM D 1510-16, preferably from 35 to 145 g/kg, for example 35, 40, 35, 50, 55, 60, 65, 70, 75, 80, 85, 90, 95, 100, 105, 110, 115, 120, 125, 130, 135, 140, or 145 g/kg as measured by the Iodine Adsorption Number determined according to ASTM D 1510-16. The Oil Absorption Number (OAN) of the carbon black may be from 60 to 180 cm³/100 g (10⁻⁵ m³/kg) as determined according to ASTM D2414, for example 60, 70, 80, 90, 100, 110, 120, 130, 140, 150, 160, 170, or 180 cm³/100 g (10⁻⁵ m³/kg) as determined according to ASTM D2414. Additionally, the carbon black may comprise one or more carbon blacks having an American Society for Testing and Materials (ASTM) designations selected from the group consisting of N134, N234, N326, N330, N550 and N660, preferably N326, N330, N550 and N660. These designations may have the following characteristics.

| ASTM designation | Particle size (nm) | Surface area (m²/g) |
|---|---|---|
| N134 | 20-25 | 135-155 |
| N234 | 24-33 | 115-135 |
| N326 | 30-40 | 90-100 |
| N330 | 28-36 | 76-80 |
| N550 | 39-55 | 39-41 |
| N660 | 56-70 | 34-36 |

The composition may further comprise one or more of the following: an antioxidant or stabiliser, for example 2,2,4-Trimethyl-1,2-dihydroquinoline polymer (TMQ) and/or N-(1,3-dimethylbutyl)-N'-phenyl-p-phenylenediamine (6PPD), preferably each in the amount of 0.5 wt% of the plasticizer absorbing material; a tackifier, for example Koresin^{®}, preferably in the amount of 1 wt% of the plasticizer absorbing material; a crosslinking or vulcanisation agent, for example sulfur, preferably in an amount of 1.5 wt% of the plasticizer absorbing material; one or more accelerators selected from the group consisting of zinc oxide, preferably in an amount of 2.5 wt% of the plasticizer absorbing material, N-cyclohexyl-2-benzothiazolesulfenamide (CBS), preferably in an amount of 1 wt% of the plasticizer absorbing material, N-tert-butyl-benzothiazole sulphonamide (TBBS), benzothiazyl disulphide (MBTS), N,N-dicyclohexyl-2-benzothiazolesulfenamide (DCBS), and tetrabenzylthiuram disulfide (TBzTD); and a dispersing agent, for example stearic acid, preferably in an amount of 1 wt% of the plasticizer absorbing material.

In embodiments that are especially preferred, the plasticizer absorbing material of the present invention may comprise (or be prepared from or using):
an elastomer present in a total amount of from 40 to 80 wt% of the plasticizer absorbing material, wherein the elastomer comprises natural rubber and one or more of butadiene rubber and styrene-butadiene rubber; and,
a reinforcing filler component present in a total amount of from 20 to 40 wt% of the plasticizer absorbing material, wherein the reinforcing filler component comprises a carbon black, preferably one or more carbon blacks having a surface area of the carbon black may be from 30 to 170 g/kg as measured by the Iodine Adsorption Number determined according to ASTM D 1510-16 and/or an Oil Absorption Number (OAN) of from 60 to 180 cm³/100 g (10⁻⁵ m³/kg) as determined according to ASTM D2414.

The plasticizer absorbing material may be located radially and/or axially outside the RF transmitter. The RF transmitter may be coated with or coated in the plasticizer absorbing material. The plasticizer absorbing material may coat at least part, and optionally the whole, of the RF transmitter. When coated, the thickness of the plasticizer absorbing material may be from 0.5 mm to 10 mm, and optionally from 0.5 mm to 5 mm, for example 0.5, 1, 1.5, 2, 2.5, 3, 3.5, 4, 4.5, or 5 mm. Typically, the lower limit of thickness is given by RF transmitter size, the upper limit is given by tire component thickness in which the RF transmitter (sensor) is embedded.

The plasticizer absorbing material is prepared by mixing the components described hereinabove and curing or vulcanising them to provide a cured product. In preparing the plasticizer absorbing material of the present invention, the method for combining each of the components is not limited and any of the methods known to the person skilled in the art may be used. For example, all of the component materials may be blended and kneaded at once or they may be blending and kneaded in multiple steps. For blending and kneading, a kneader such as roll kneader, internal mixer or Banbury mixer may be used.

The RF transmitter may be disposed between two layers of the plasticizer absorbing material to produce a rubberized sandwich structure. The sandwich is then embedded in the desired part of the tire during tire assembly on the tire assembly machine. The sandwich structure may be prepared and embedded in a tyre according to the methods described in WO2023034676A1.

The curing/vulcanization conditions for curing the plasticizer absorbing material are not limited and can be any of those known to the person skilled in the art. Typically, however, vulcanization conditions of treatment at 140 to 180° C for 5 to 120 minutes are employed.

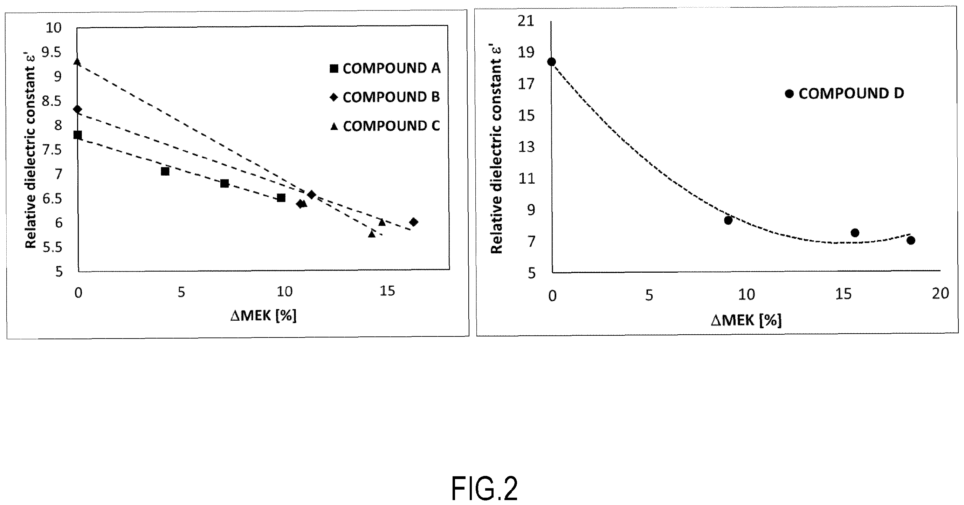

Fig. 2 illustrates the way different plasticizer absorbing materials are altered by a plasticizer (in this case, octyl oleate). The left hand graph shows three different curves for three different plasticizer absorbing materials (namely, Compound A, Compound B and Compound C). It can be seen that Compound A has the lowest relative dielectric constant initially (i.e. at 0% absorbed plasticizer) at around 7.7, compared to about 8.2 and 9.3 for Compounds B and C, respectively. It can also be seen that the relative dielectric constant of Compounds A and B drop at a similar rate, whereas that of Compound C drops at a faster rate. Compound C therefore will give a larger change in signal strength or reading distance, and so may be better candidate for use in monitoring plasticizer migration.

However, in any case, it is preferable to select a plasticizer absorbing material composition whose relative dielectric constant response vs migrated oil content is generally linear and generally sloping within the concentration range of interest.

Compounds A-C each a have generally linear and sloping response as the plasticizer absorption increases in the range of plasticizer absorption shown.

The right hand graph shows a curve for a single plasticizer absorbing material (Compound D). Compound D's response to plasticizer migration is non-linear when considered over a range of 0% to about 18% plasticizer, but is generally linear (and sloping) in the smaller range of 0% to 5% plasticizer. Compound D also has a much higher relative dielectric constant initially (around 18.4) and also drops much faster than Compounds A-C initially (dropping by around 10 between 0% and 10% absorption, compared to a drop of between 1.4 and 2.5 for compounds A-C).

The constituents of Compounds A-D are given in Table 1 below.

**Table 1**

| **Material %** | **Compound A** | **Compound B** | **Compound C** | **Compound D** |
|---|---|---|---|---|
| Natural Rubber | 45 | 50 | 40 | 55 |
| Butadiene Rubber | | 15 | | |
| Styrene Butadiene rubber | 20 | | 25 | |
| CB N330 (mid surface area & high structu re) | | 8 | 26 | 33 |
| CB N550 (mid surface area, low structu re) | | 18 | | |
| CB N660 (low surf area, low structure) | 27 | | | |
| TMQ | 0.5 | 0.5 | 0.5 | 0.5 |
| 6PPD | 0.5 | 0.5 | 0.5 | 0.5 |
| KORESIN | 1 | 1 | 1 | 1 |
| SULFUR | 1.5 | 2.5 | 2.5 | 4 |
| Zinc Oxide | 2.5 | 2.5 | 2.5 | 4 |
| Stearic Acid | 1 | 1 | 1 | 1 |
| CBS | 1 | 1 | 1 | 1 |

The plasticizer is not limited in the present invention and may be any known to the skilled person. The plasticizer is a liquid plasticizer that may be selected from the group consisting of liquid diene polymers, polyolefinic oils, naphthenic oils, paraffinic oils, DAE (Distillate Aromatic Extracts) oils, MES (Medium Extracted Solvates) oils, TDAE oils are particularly suitable. (Treated Distillate Aromatic Extracts), RAE oils (Residual Aromatic Extract oils), TRAE oils (Treated Residual Aromatic Extract), SRAE oils (Safety Residual Aromatic Extract oils), mineral oils, vegetable oils, ether plasticizers, ester plasticizers, phosphate plasticizers, sulfonate plasticizers and mixtures of these compounds. For example, the liquid plasticizer may be octyl oleate, such as Plasthall^{®} 425.

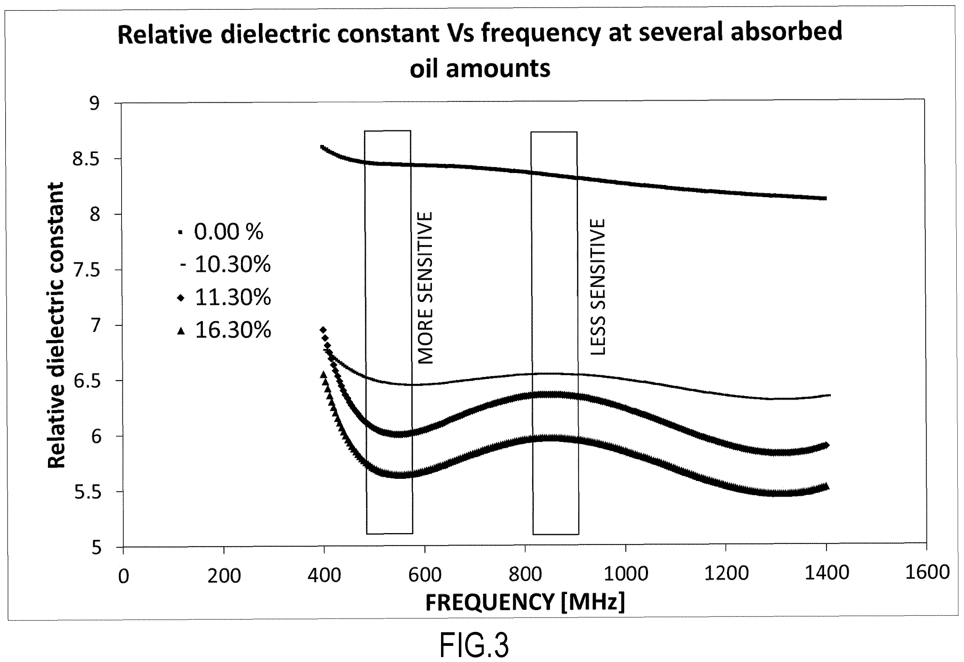

Fig. 3 illustrates how the relative dielectric constant of a particular plasticizer absorbing material (in this case Compound B) varies according to frequency of the RF signal for different absorbed plasticizer contents. Four different curves are shown for different plasticizer contents. For example, the top curve shows 0% plasticizer, and the curve drops (showing a drop in relative dielectric constant) as the frequency increases from 400MHz to 1400MHz.

It can be seen that certain frequency ranges give better sensitivity to a change in plasticizer content. For example, the right hand box covers a range of about 825-900MHz and this range is less sensitive because the curves are closer together. On the other hand, the left hand box which covers a range of about 475-550MHz is more sensitive because the curves are further apart. Therefore, selecting a frequency within that more sensitive range is likely to be preferable in terms of showing plasticizer migration.

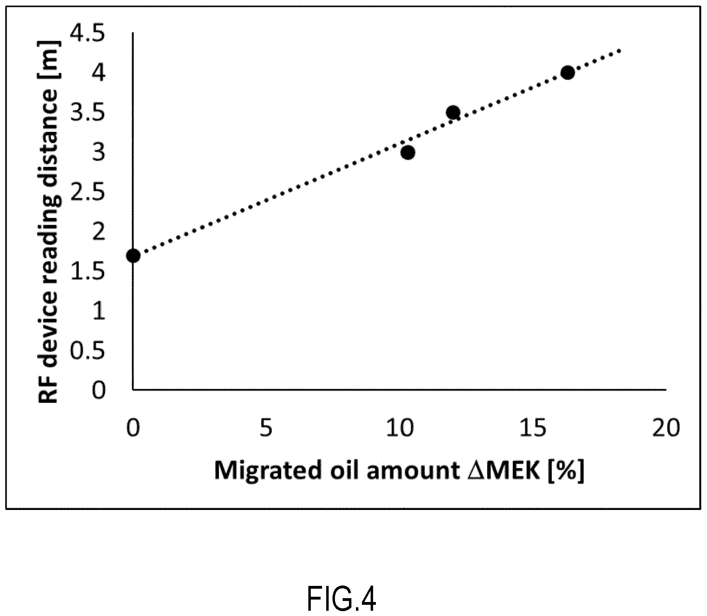

Fig. 4 shows how, for a particular plasticizer absorbing material (Compound B), the RF signal reading distance varies according to amount of absorbed plasticizer. In particular, when the amount of absorbed plasticizer is 0%, the reading distance is about 1.7m. As the amount of absorbed plasticizer increases, the reading distance increases linearly from about 1.7m at about 0% to about 4.2m at about 18%.

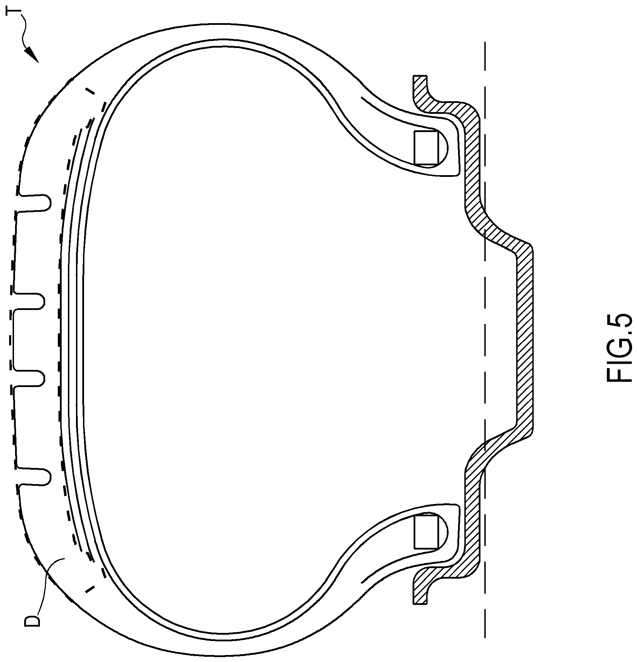

Fig. 5 is a cross-section of a tyre T along an axial-radial plane. The tyre includes an area D shown dotted in the figure, which indicates the preferred positions for locating the RFID tag and plasticizer absorbing material. The dotted area D covers a) the area of the tyre between the tread and cap plies (if cap plies are present) of the tyre and the tread outer surface which is axially within the width of the tread and cap plies, and b) the area of the tyre which is axially outside the width of the tread and cap plies within a buttress area of the tyre and between a radially outermost body ply of the tyre and the tyre outer surface. Area a) corresponds generally to the tread area of the tyre, and b) corresponds generally to the buttress area of the tyre. Locating the tag and plasticizer absorbing material in these areas means that plasticizer migration in those areas can be reliably monitored.

Area a), which is part of area D in Fig. 5, covers both a higher plasticizer content region of the tyre (the tread compound) as well as a lower plasticizer content region of the tyre (the base compound).

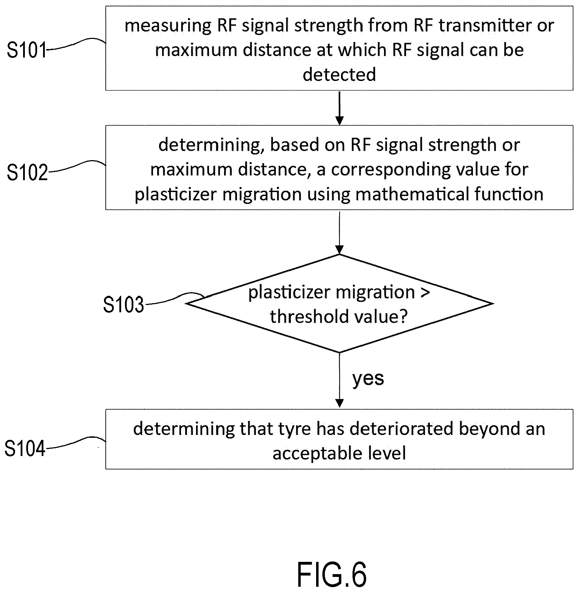

Fig. 6 is a flowchart illustrating a first example method of using the RFID tag and plasticizer absorbing material to determine plasticizer migration in the tyre. In a first step S101, an RF receiver (e.g. an RF reader) is used to measure a signal strength from the RFID tag. The RF receiver is located at a known distance from the tyre (for example, 1m away). For example, the RF receiver may be fixed in position, such as when mounted on a vehicle, and so the distance from the tyre does not change, and is known.

In the second step S102, using the fact that the relationship of how the signal strength varies with absorbed plasticizer content at a distance of 1m is known, the measured signal strength can be used to determine a corresponding value of absorbed plasticizer content.

In the third step S103, the value of absorbed plasticizer content is compared with a threshold value. If the value exceeds the threshold value, in the fourth step S104 it is determined that the tyre has deteriorated beyond an acceptable level. If the method is being carried out on computer system, for example in the ECU of a vehicle, the computer system may warn the driver that the tyre has deteriorated beyond an acceptable level and ought to be changed.

Alternatively in the first step S101, when the position of the RF receiver is not known, it may be necessary to acquire information on the position of the RF receiver (and this information may be acquired by measurement of the position by the RF receiver if the RF receiver is capable of doing so) as well as measurement of the RF signal strength. The measured signal strength and acquired information on the position of the RF receiver are then used in step S102 to determine a corresponding value of absorbed plasticizer content. This may be done by using the relationship of how the signal varies with absorbed plasticizer content at the position of the RF receiver, to determine the absorbed plasticizer content for the measured signal strength.

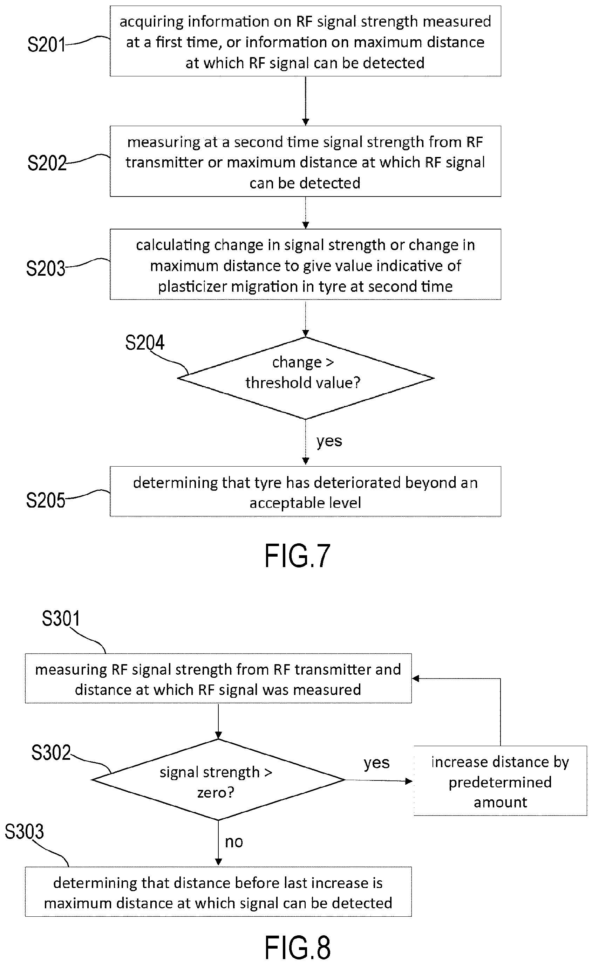

Alternatively again in the first step S101, the maximum distance at which an RF signal can be detected may be measured, which is explained in more detail below with reference to Fig. 8. In step S102, using the maximum distance, a corresponding value for plasticizer migration is determined, which can then be compared to a threshold value in step S103.

Fig. 7 is a flowchart illustrating a second example method of using the RFID tag and plasticizer absorbing material to determine plasticizer migration in the tyre. In a first step S201, information on an RF signal strength measured at a first time is acquired. For example, in the first step S201 an RF receiver (e.g. an RF reader) may be used to measure a signal strength from the RFID tag. Alternatively, the signal strength may have been measured previously and in step S201 the information is simply acquired, for example, from a memory.

In a second step S202, the RF receiver is used to measure a signal strength from the RFID tag at a second time. The second time may be several months or years after the first time.

At the first time and the second time, the distance of the RF receiver from the tyre is fixed (for example, 1m away) and the RF receiver may be mounted on a vehicle.

In a third step S203, the change in signal strength is calculated to give a value indicative of plasticizer migration in the tyre at the second time.

In fourth step S204, the value indicative of plasticizer migration is compared with a threshold value. If the value exceeds the threshold value, in the fifth step S205 it is determined that the tyre has deteriorated beyond an acceptable level. If the method is being carried out on computer system and for example in the ECU of a vehicle, the computer system may warn the driver that the tyre has deteriorated beyond an acceptable level and ought to be changed.

Alternatively, when the position of the RF receiver is not fixed at the first and second time, in the first step S201 and the second step S202 it may be necessary to acquire information on the position of the RF receiver (and this information may be acquired by measurement of the position by the RF receiver if the RF receiver is capable of doing so). This information may be used to determine when the positions of the RF receiver at the first and second times are the same, and so when it is acceptable to compare the signal strengths.

Alternatively again in the first step S201 and the second step S202, information on the maximum distance at which RF signal can be detected may be acquired, which is explained in more detail below with reference to Fig. 8. Then, in step S203, using the maximum distances, the change in maximum distance is calculated to give a value indicative of plasticizer migration in the tyre at the second time.

Fig. 8 shows how the maximum distance at which an RF signal can be detected may be measured. In short, the signal strength is measured and distance is increased stepwise until the signal strength becomes zero. The last distance at which the signal was non-zero is determined to be the maximum distance at which RF signal can be detected.

As used in this application, the terms "component," "module," "engine," "system," "apparatus," "interface," or the like are generally intended to refer to a computer-related entity, either hardware, a combination of hardware and software, software, or software in execution. For example, a component may be, but is not limited to being, a process running on a processor, a processor, an object, an executable, a thread of execution, a program, and/or a computer. By way of illustration, both an application running on a controller and the controller can be a component. One or more components may reside within a process and/or thread of execution and a component may be localized on one computer and/or distributed between two or more computers.

Furthermore, the claimed subject matter may be implemented as a method, apparatus, or article of manufacture using standard programming and/or engineering techniques to produce software, firmware, hardware, or any combination thereof to control a computer to implement the disclosed subject matter. For instance, the claimed subject matter may be implemented as a computer-readable medium embedded with a computer executable program, which encompasses a computer program accessible from any computer-readable storage device or storage media. For example, computer readable media can include but are not limited to magnetic storage devices (e.g., hard disk, floppy disk, magnetic strips), optical disks (e.g., compact disk (CD), digital versatile disk (DVD)), smart cards, and flash memory devices (e.g., card, stick, key drive).

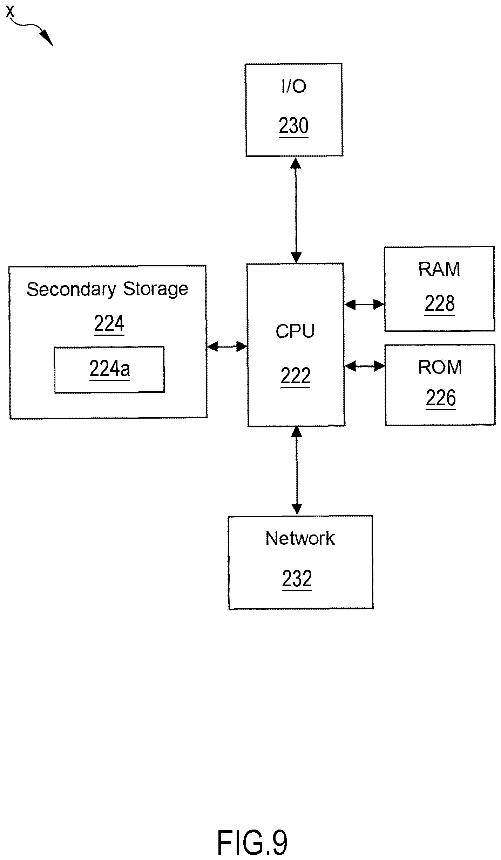

Fig. 9 is a block diagram showing a technical architecture of a server X. The server X may include a computer system configured to carry out a method of the present invention, and may include an RF receiver.

The technical architecture includes a processor 222 (which may be referred to as a central processor unit or CPU) that is in communication with memory devices including secondary storage 224 (such as disk drives), read only memory (ROM) 226, random access memory (RAM) 228. The processor 222 may be implemented as one or more CPU chips. The technical architecture may further comprise input/output (I/O) devices 230, and network connectivity devices 232.

The secondary storage 224 is typically made up of one or more disk drives or tape drives and is used for non-volatile storage of data and as an over-flow data storage device if RAM 228 is not large enough to hold all working data. Secondary storage 224 may be used to store programs which are loaded into RAM 228 when such programs are selected for execution.

In this embodiment, the secondary storage 224 has an order processing component 224a comprising non-transitory instructions operative by the processor 222 to perform various operations of the method of the present disclosure. The ROM 226 is used to store instructions and perhaps data which are read during program execution. The secondary storage 224, the RAM 228, and/or the ROM 226 may be referred to in some contexts as computer readable storage media and/or non-transitory computer readable media.

I/O devices 230 may include printers, video monitors, liquid crystal displays (LCDs), plasma displays, touch screen displays, keyboards, keypads, switches, dials, mice, track balls, voice recognizers, card readers, paper tape readers, or other well-known input devices.

The network connectivity devices 232 may take the form of modems, modem banks, Ethernet cards, universal serial bus (USB) interface cards, serial interfaces, token ring cards, fiber distributed data interface (FDDI) cards, wireless local area network (WLAN) cards, radio transceiver cards that promote radio communications using protocols such as code division multiple access (CDMA), global system for mobile communications (GSM), long-term evolution (LTE), worldwide interoperability for microwave access (WiMAX), near field communications (NFC), radio frequency identity (RFID), and/or other air interface protocol radio transceiver cards, and other well-known network devices. These network connectivity devices 232 may enable the processor 222 to communicate with the Internet or one or more intranets. With such a network connection, it is contemplated that the processor 222 might receive information from the network, or might output information to the network in the course of performing the above-described method operations. Such information, which is often represented as a sequence of instructions to be executed using processor 222, may be received from and outputted to the network, for example, in the form of a computer data signal embodied in a carrier wave.

The processor 222 executes instructions, codes, computer programs, scripts which it accesses from hard disk, floppy disk, optical disk (these various disk based systems may all be considered secondary storage 224), flash drive, ROM 226, RAM 228, or the network connectivity devices 232. While only one processor 222 is shown, multiple processors may be present. Thus, while instructions may be discussed as executed by a processor, the instructions may be executed simultaneously, serially, or otherwise executed by one or multiple processors.

Although the technical architecture is described with reference to a computer, it should be appreciated that the technical architecture may be formed by two or more computers in communication with each other that collaborate to perform a task. For example, but not by way of limitation, an application may be partitioned in such a way as to permit concurrent and/or parallel processing of the instructions of the application. Alternatively, the data processed by the application may be partitioned in such a way as to permit concurrent and/or parallel processing of different portions of a data set by the two or more computers. In an embodiment, virtualization software may be employed by the technical architecture 220 to provide the functionality of a number of servers that is not directly bound to the number of computers in the technical architecture 220. In an embodiment, the functionality disclosed above may be provided by executing the application and/or applications in a cloud computing environment. Cloud computing may comprise providing computing services via a network connection using dynamically scalable computing resources. A cloud computing environment may be established by an enterprise and/or may be hired on an as-needed basis from a third party provider.

It is understood that by programming and/or loading executable instructions onto the technical architecture, at least one of the CPU 222, the RAM 228, and the ROM 226 are changed, transforming the technical architecture in part into a specific purpose machine or apparatus having the novel functionality taught by the present disclosure. It is fundamental to the electrical engineering and software engineering arts that functionality that can be implemented by loading executable software into a computer can be converted to a hardware implementation by well-known design rules.

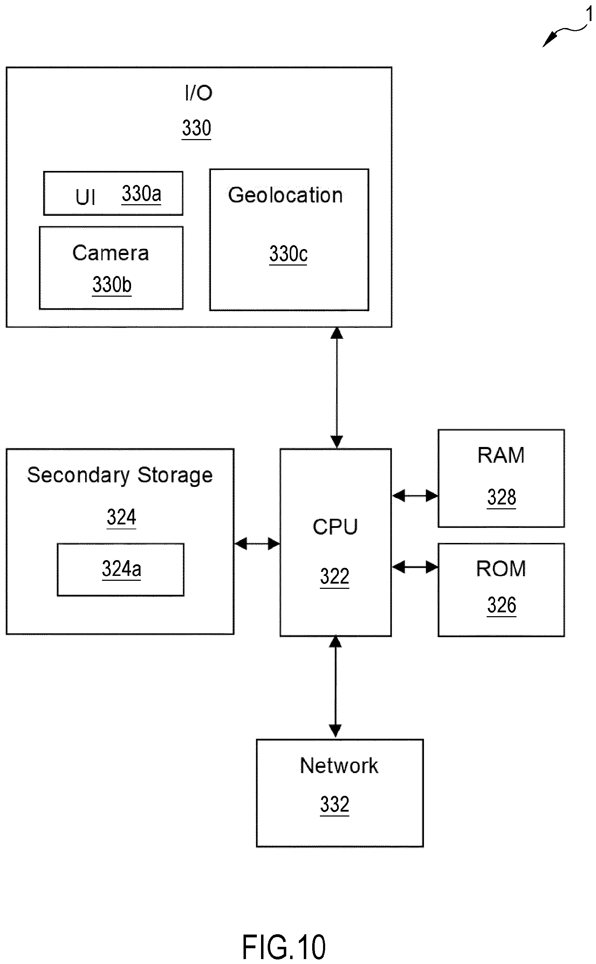

Fig. 10 is a block diagram showing a technical architecture of a communication device 1. It is envisaged that in embodiments, the communication device 1 will be a smartphone or tablet device. The communication device 1 (e.g. a smartphone or tablet device) may include a computer system configured to carry out a method of the present invention, and may include an RF receiver (e.g. the smartphone or tablet device may include the RF receiver).

The technical architecture includes a processor 322 (which may be referred to as a central processor unit or CPU) that is in communication with memory devices including secondary storage 324 (such as disk drives or memory cards), read only memory (ROM) 326, random access memory (RAM) 328. The processor 322 may be implemented as one or more CPU chips. The technical architecture further comprises input/output (I/O) devices 330, and network connectivity devices 332.

The I/O devices comprise a user interface (Ul) 330a, a camera 330b and a geolocation module 330c. The UI 330a may comprise a touch screen, keyboard, keypad or other known input device. The camera 330b allows a user to capture images and save the captured images in electronic form. The geolocation module 330c is operable to determine the geolocation of the communication device using signals from, for example global positioning system (GPS) satellites.

The secondary storage 324 is typically made up of a memory card or other storage device and is used for non-volatile storage of data and as an over-flow data storage device if RAM 328 is not large enough to hold all working data. Secondary storage 324 may be used to store programs which are loaded into RAM 328 when such programs are selected for execution.

In this embodiment, the secondary storage 324 has an order generation component 324a, comprising non-transitory instructions operative by the processor 322 to perform various operations of the method of the present disclosure. The ROM 326 is used to store instructions and perhaps data which are read during program execution. The secondary storage 324, the RAM 328, and/or the ROM 326 may be referred to in some contexts as computer readable storage media and/or non-transitory computer readable media.

The network connectivity devices 332 may take the form of modems, modem banks, Ethernet cards, universal serial bus (USB) interface cards, serial interfaces, token ring cards, fiber distributed data interface (FDDI) cards, wireless local area network (WLAN) cards, radio transceiver cards that promote radio communications using protocols such as code division multiple access (CDMA), global system for mobile communications (GSM), long-term evolution (LTE), worldwide interoperability for microwave access (WiMAX), near field communications (NFC), radio frequency identity (RFID), and/or other air interface protocol radio transceiver cards, and other well-known network devices. These network connectivity devices 332 may enable the processor 322 to communicate with the Internet or one or more intranets. With such a network connection, it is contemplated that the processor 322 might receive information from the network, or might output information to the network in the course of performing the above-described method operations. Such information, which is often represented as a sequence of instructions to be executed using processor 322, may be received from and outputted to the network, for example, in the form of a computer data signal embodied in a carrier wave.

The processor 322 executes instructions, codes, computer programs, scripts which it accesses from hard disk, floppy disk, optical disk (these various disk based systems may all be considered secondary storage 324), flash drive, ROM 326, RAM 328, or the network connectivity devices 332. While only one processor 322 is shown, multiple processors may be present. Thus, while instructions may be discussed as executed by a processor, the instructions may be executed simultaneously, serially, or otherwise executed by one or multiple processors.

Preferred embodiments of the invention have been described purely by way of example, and various modifications, additions and/or omissions will present themselves to one skilled in the art, all of which form part of the invention.

## Claims

1. A tyre having a higher plasticizer content region and a lower plasticizer content region, the tyre having integrated in it an RF transmitter and a plasticizer absorbing material configured to absorb migrated plasticizer,
wherein the relative dielectric constant of the plasticizer absorbing material when the tyre is in a cured state is ≥6.5.

2. A tyre having a higher plasticizer content region and a lower plasticizer content region, the tyre having integrated in it an RF transmitter and a plasticizer absorbing material configured to absorb migrated plasticizer,
wherein, for each unit% of adsorbed plasticizer amount, the relative dielectric constant of the plasticizer absorbing material changes in an absolute value range of from 0.1 to 2.

3. A tyre according to claim 1 or 2, wherein the plasticizer absorbing material coats at least part of the RF transmitter.

4. A tyre according to any preceding claim, wherein the relative dielectric constant is in this range or changes in this range at a frequency from 10 MHz to 1600 MHz, optionally at a frequency from 400 MHz to 1000 MHz.

5. A tyre according to any preceding claim, wherein the RF transmitter and/or plasticizer absorbing material are located either a) between any tread and cap plies of the tyre and the tread outer surface while being located axially within the width of any tread and cap plies, or b) axially outside the width of any tread and cap plies and within a buttress area of the tyre and between a radially outermost body ply of the tyre and the tyre outer surface.

6. A tyre according to any preceding claim, wherein the plasticizer absorbing material comprises:
an elastomer present in a total amount of from 40 to 80 wt% of the plasticizer absorbing material, wherein the elastomer comprises natural rubber and one or more of butadiene rubber and styrene-butadiene rubber; and,
a reinforcing filler component present in a total amount of from 20 to 40 wt% of the plasticizer absorbing material, wherein the reinforcing filler component comprises a carbon black, preferably one or more carbon blacks having a surface area of the carbon black may be from 30 to 170 g/kg as measured by the Iodine Adsorption Number determined according to ASTM D 1510-16 and/or an Oil Absorption Number (OAN) of from 60 to 180 cm³/100 g (10⁻⁵ m³/kg) as determined according to ASTM D2414.

7. An RF device for embedding within a tyre, the RF device comprising an RF transmitter and a plasticizer absorbing material coating at least part of the RF transmitter for absorbing migrated plasticizer from a tyre,
wherein the relative dielectric constant of the plasticizer absorbing material is ≥6.5, and/or
wherein, for each unit% of adsorbed plasticizer amount, the relative dielectric constant of the plasticizer absorbing material changes in an absolute value range of from 0.1 to 2.

8. A method of determining plasticizer migration in a tyre, the tyre having a higher plasticizer content region and a lower plasticizer content region and an RF transmitter integrated in the tyre, the method comprising:
measuring, using an RF receiver, an RF signal strength from the RF transmitter, or measuring, using an RF receiver, an RF signal strength from the RF transmitter and acquiring information on the position at which the RF signal strength was measured; and
based on the measured signal strength or the position at which the RF signal strength was measured, determining a corresponding value for plasticizer migration using information on how the signal strength or measured position varies with plasticizer migration for that tyre type.

9. A method of determining plasticizer migration in a tyre, the tyre having a higher plasticizer content region and a lower plasticizer content region and an RF transmitter integrated in the tyre, the method comprising:
acquiring information on an RF signal strength measured at a first time, or information on an RF signal strength measured at a first time and the position at which the RF signal strength was measured;
measuring using an RF receiver an RF signal strength from the RF transmitter at a second time, or measuring using an RF receiver an RF signal strength from the RF transmitter at a second time and acquiring information on the position at which the RF signal strength was measured, and
comparing the signal strengths or the positions where the measurements are taken at the first time and the second time, to give a value indicative of plasticizer migration in the tyre at the second time.

10. A method according to claim 9, further comprising comparing the value indicative of plasticizer migration with a threshold value and, if the value is greater than or less than the threshold value, determining that the tyre has deteriorated beyond an acceptable level.

11. A tyre condition monitoring apparatus, comprising:
an RF receiver; and
a computer system containing a processor and a memory storing program instructions operative to cause the computer to execute the steps of the method of claim 8, 9 or 10.

12. Tyre condition monitoring apparatus according to claim 11, wherein computer system is provided by the ECU of a vehicle.

13. A tyre condition monitoring system comprising a tyre condition monitoring apparatus according to claim 11 or 12 and a tyre, wherein the tyre has a higher plasticizer content region and a lower plasticizer content region, and an RF transmitter integrated in the tyre.

14. A tyre condition monitoring system according to claim 13, wherein the tyre is according to any one of claims 1 to 6.

15. A computer program product comprising instructions, which, when the program is executed by a computer, cause the computer to implement a method according to claim 8, 9 or 10.
